# EUROPEAN PATENT SPECIFICATION

(11) **EP 0 921 772 B1**
(45) Date of publication and mention of the grant of the patent: **26.03.2003**
(21) Application number: 98914212.0
(22) Date of filing: 02.04.1998
(51) Int. Cl.: A61F 2/76, A61F 2/62

(54) **ADJUSTABLE ADAPTER FOR A LOWER LEG PROSTHESIS**
EINSTELLBARER ADAPTER FÜR EINE UNTERSCHENKELPROTHESE
ADAPTATEUR AJUSTABLE DESTINE A UNE PROTHESE DE JAMBE INFERIEURE

(30) Priority: 02.04.1997 SE 9701218
(43) Date of publication of application: 16.06.1999
(73) Proprietor: GRAMTEC INNOVATION AB, S-511 56 Kinna (SE)
(72) Inventor: GRAMNÄS, Finn, S-511 56 Kinna (SE)
(74) Representative: Hammond, Andrew David
(86) International application number: SE9800612
(87) International publication number: WO98043559

(56) References cited:
- DE-A- 3 937 379
- FR-A- 2 410 998
- US-A- 5 047 063
- US-A- 5 326 352

## Description

The present invention pertains to an adjustable adapter especially for lower leg prosthesis including an adapter housing attachable to the lower leg prosthesis, a spheroidal adapter head mounted movably therein and adjustable by adjusting means of the adapter housing, and a prosthesis collar attachment member that can be shifted sideways relative to the adapter head, which latter is attachable to a prosthesis collar, and by means of a connector is attachable to the adapter housing, at which the spheroidal part of the adapter head, in mounted position, is integrated with the adapter housing forming a unit assembled by two mutually movable parts which are fixable in different positions by means of the adjusting means, independantly of the adjustability and attachment of the adapter head to the prosthesis collar attachment member, at which in the adapter head there is arranged an elongted through groove, through which said connector is arranged to extend and which permits a transverse movement between the adapter head and the prosthesis collar attachment member, independantly of the adjustability of the adapter housing the relative to the adapter head.

Prostheses for the knee, lower leg or foot are normally fastened to the bearer's bone using a prosthesis collar. To make the prosthesis adjustable at the desired angle and to make it possible to adjust it transversely to the prosthesis collar adapters for that purpose have previously been proposed.

US-A-5 013 325 (SE-B-8602143-3) describes a lower leg prosthesis equipped with such an adapter which includes a prosthesis collar attachment member for attachment to the lower collar of the prosthesis. On the side facing the prosthesis collar the prosthesis collar attachment member is screwed to an adapter element including a little hemisphere around the screw shaft, which hemisphere is made rotatable against a corresponding spheroidal inner surface in an adapter housing which is attached to a lower leg prosthesis at its opposite end. At a distance from the hemisphere there is a concentrically attached disc against which the prosthesis collar attachment abuts. Further, there are external bosses on the adapter housing equipped with threaded penetrating holes at an angle to the central axis in which pinning screws can be screwed in from below so that their front ends press against the disc attached to the hemisphere thus pressing the adapter housing to different angular positions relative to the adapter housing. The prosthesis collar attachment element has a penetrating hole of significantly greater dimensions than the fastening screws in it whereby using a washer it is possible to attach the prosthesis collar attachment element in different sideways adjustable positions relative to the adapter housing. Using this design it is possible to achieve different angular positions without detaching the prosthesis, but because of the small radius of the sphere the angular variation is relatively limited. At the same time a sideways adjustment can be achieved but for this it is necessary that the whole prosthesis including the prosthesis collar be detached to reach the side adjustment screws, all of which makes fitting more difficult.

Other types of similar adapters have the two adjustment functions separated to such a degree that the designs are large and unwieldy.

The purpose of the present invention is to provide an adjustable adapter for a lower leg prosthesis for which the above disadvantages are essentially eliminated, which has been achieved by giving the adapter the characteristics described in the characterizing parts of the patent claims.

In the following the invention will be described in greater detail with reference to the embodiments illustrated schematically in the attached figures.

Figs. 1 - 6g do not represent the invention according to claim 1.
Fig. 1 shows an embodiments of a lower leg prosthesis with included adapter in schematic cross section from the side.
Fig. 2 is a side view of the adapter according to Fig. 1.
Fig. 3 shows an exploded view from the side of the adapter according to Fig. 2.
Fig. 4 is an exploded view corresponding to Fig. 3 which shows the adapter's most important parts in section.
Fig. 5 shows an adapter head included in the adapter in a view from above.
Fig. 6a-g show different adjustments of the adapter in side views seen in section.
Fig. 7 shows a section through an adapter according to the invention in a locked position.
Fig. 8 shows the adapter according to Fig. 7 turned 90 degrees in a released position.

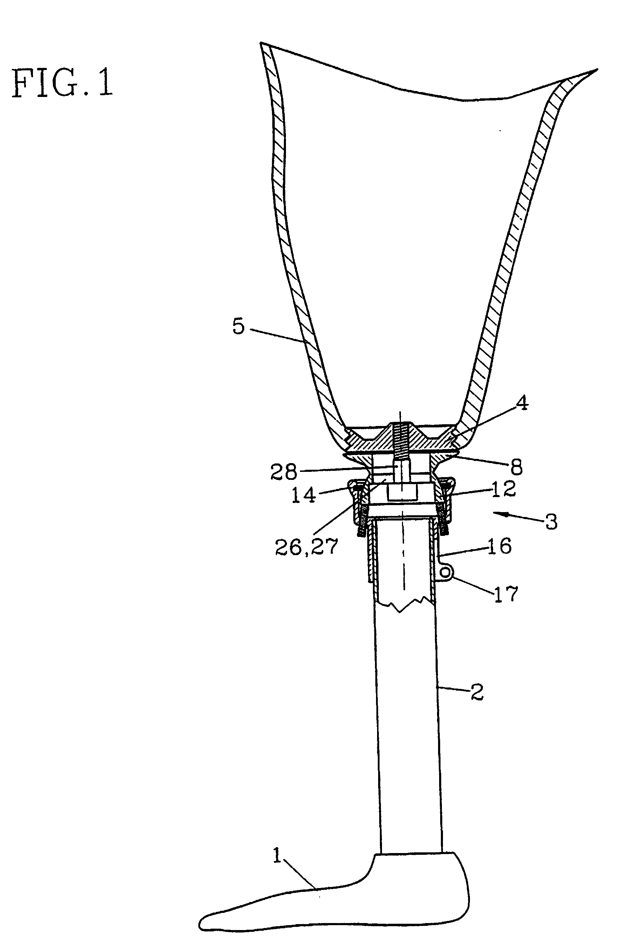

In Fig. 1 a prosthesis foot 1 with an adjacent long leg like element, preferably a tube 2, appropriately made of aluminum or another light strong material, is shown schematically. On the end facing away from the prosthesis foot the leg like element 2 is attached to an adapter 3 which is affixed to a prosthesis collar 5 using a prosthesis collar attachment member 4 that can be adjusted relative to the attachment end of the leg like element.

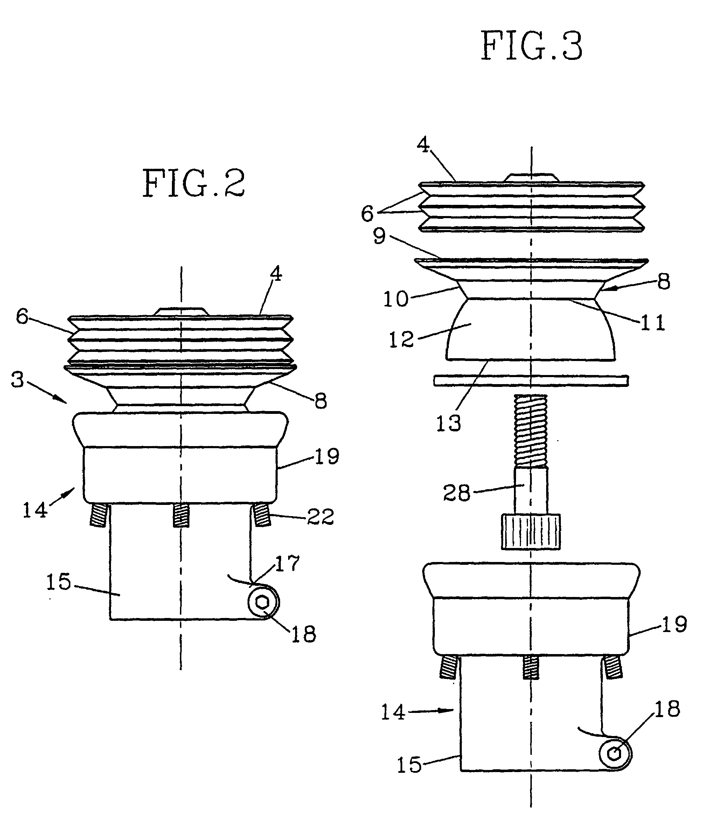

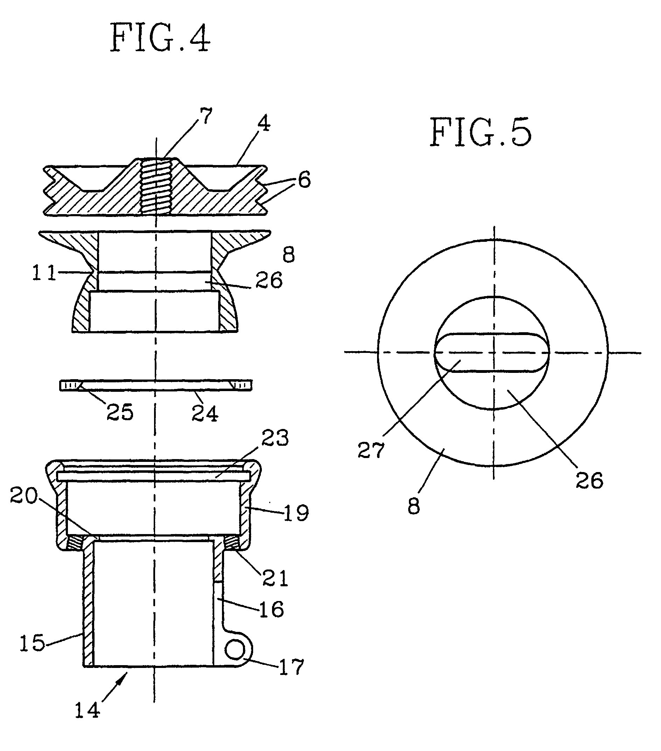

Fig. 2 to 4 show the adapter according to Fig. 1 on a larger scale from the side in an exploded view and in an exploded view in section, respectively. It is clear from the figures that the previously mentioned prosthesis collar attachment member 4 is included in the adapter 3, which member in the shown design consists of a circular disc with external grooves 6 which facilitate attachment of the member 4 to the prosthesis collar. In the shown design the disc has a central threaded hole 7 (see Fig. 1 and Fig. 4). For reasons to be given shortly, the prosthesis collar attachment member 4 or the central hole 7 can include an oblong hole (not shown). Further included in the adapter 3 is an adapter head 8 consisting of a collar with a circular ring shaped end surface 9 facing toward the prosthesis collar attachment member 4 and pressing against it. On the end surface 9 there is a conical section 10 which from a midline 11 is transformed to an essentially spherical concentric form 12. The spherical part 12 of the adapter ends in a circular ring shaped surface 13 which is mainly parallel to the adapter head's opposite end surface 9. Further there is an adapter housing 14 which includes a tube shaped section 15 designed to be attached to the tube like structure, for which purpose the under part of which has a slit 16 (see for example Fig. 1) while the material on either side of the slit 16 has attachment members 17 for a connector 18, for example a bolt, with which the end of the tube shaped part, which is somewhat elastic because of the slit, can be fastened to the upper end of the tube like structure 2. On the end facing away from the slit part of the tube shaped section 15 of the adapter housing 14 the tube like structure meets a bowl shaped sleeve 19 concentric with the tube shaped section 15 and of a larger diameter than the latter.

The sleeve 19 and the tube shaped section meet each other at a ring shaped connector 20 attached essentially perpendicular to the adapter housing's central axis. Through that connector extend a number, preferably four, equidistant angled threaded holes 21 designed for pinning screws 22 or the like. On the end of the collar 19 away from the tube shaped section 15 the collar has an encircling groove for a lock washer 24 with a hole 25 preferably of a form complementary to that of the spheroidal form 12 and with a contact line which, when assembled symmetrically, is in the middle of the spheroidal surface 12, that is, with its smaller opening turned axially out from the collar part when assembled, so that because of the increasing diameter of the spheroidal part 12 in the downward direction, after insertion into the sleeve 19 and attachment of the lock washer 24 the adapter head cannot be extracted from the sleeve but using the pinning screws it can be locked at a desired angle. The lock washer 24 is preferably equipped with a slit (not shown) which facilitates its insertion into the groove 23.

The inner diameter of the opening in the sleeve 19 is somewhat larger that the outer diameter of the end surface 13 of the adapter head's spheroidal part 12 so that it fits into the sleeve and at its ring shaped end surface presses against the pinning screws 22 in the adapter housing 14, as for instance is seen in Fig. 1 where the adapter head's middle part 11 is above the sleeve 19. By inserting the lock washer 24 into the groove 23 in the adapter housing 24 after inserting the adapter head 8 into it the lock washer prevents the adapter head from being withdrawn from the sleeve 19 while at the same time because of the spheroidal part 12 the adapter head can be set at an angle relative to the lengthwise axis of the adapter housing. The angled hole 25 in the lock washer 24 thus also contributes to making that angular positioning smooth and frictionless without scratching contact between the spheroidal part 12 and the edge of the hole 25.

In Fig. 4 it is clear from the section of the adapter head 8 that approximately at the level of the external middle part 11 it has an interior partition which has a penetrating oval hole 27 placed symmetrically on the center of the interior partition 26, as is clear from Fig. 5 in which the adapter head is shown in a top view.

For internal connection between the prosthesis collar attachment member 4 and the adapter head 8 a connector 28 is used, in the example shown in the form of a bolt through the oval hole 27 in the adapter head with its threaded shaft screwed into the threaded hole 7 in the prosthesis collar attachment member 4. Because the connector 28 has a part that cannot go through the oval hole 27, that is, in the present case the head of the boit is larger in diameter that the width of the hole 27, the connector can be applied without its head going through the hole 27. Because that hole is oval it is still possible to attach the connector 28 in different positions along the length of the hole making adjustment along the length of the hole possible. With the alternative design of the prosthesis collar attachment member with an oblong hole instead of the centered hole 7 using a slide groove (not shown) in the prosthesis collar attachment member it is possible to use a nut as the connector 28 which is self locking along the length of the hole in the prosthesis collar attachment member 4. Thus the extent of the adjustability of the prosthesis adapter is further increased.

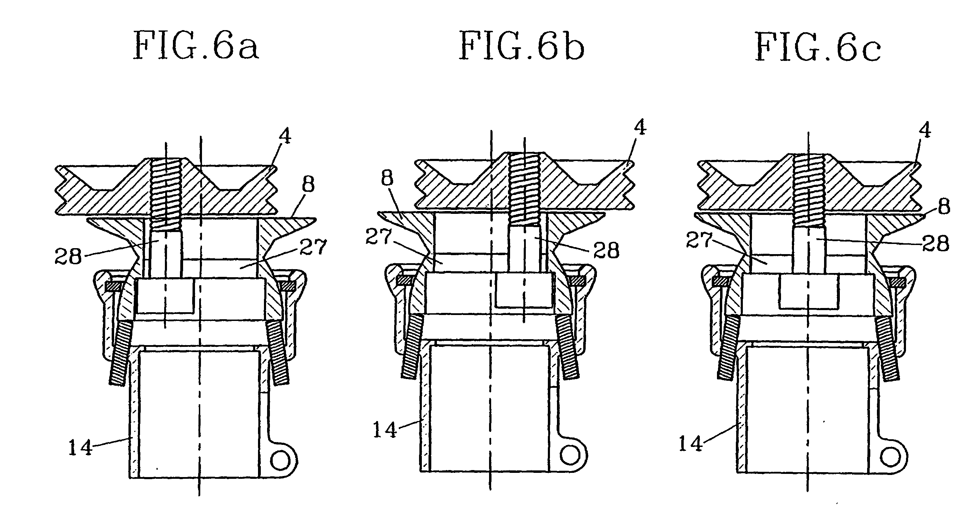

In Fig. 6a-g the adapter 3 is shown in section in different adjustment positions between the prosthesis collar attachment member 4 and the adapter head 8 and between the adapter head 8 and the adapter housing 14. In this way a great variety of adjustment positions is made possible which further can be realized with an adapter that can be of a low and particularly compact design because of the large spheroidal part of the adapter head in the sleeve of the adapter housing.

Thus Fig. 6a shows how the adapter head 8 is adjusted so that its center line forms a direct extension of the center line of the adapter housing 14, that is, no angular displacement of the center line results. On the other hand the axis of the prosthesis collar attachment member 4 is shifted parallel with respect to the center line of the adapter head 8 and therefore to the center axis of the adapter housing. This has been achieved by shifting the connector 28 to its position, in the figure, furthest to the left in the oval hole 27 in the adapter head.

Also in Fig. 6b and 6c the adapter head 8 and the adapter housing are arranged with no angle between them while the prosthesis collar attachment member 4 in Fig. 6b is placed at its other maximally shifted end position and the connector 28 in Fig. 6c is placed centrally in the hole 27 so that the center line of the prosthesis collar attachment member 4 not only is parallel to but also coincides with the center line of the adapter housing 14 and of the adapter head 8.

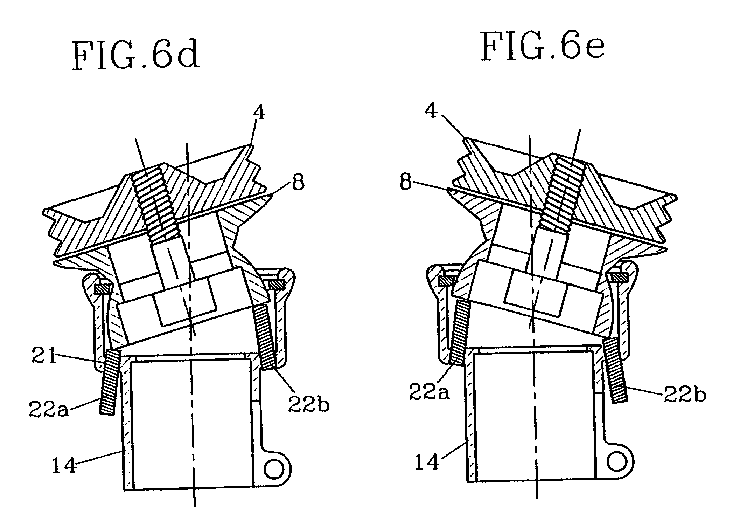

In Fig. 6d and 6e is shown how the prosthesis collar attachment member 4 and the adapter head 8 are in the same relative position as shown in Fig. 6c but where in both cases the adapter head 8 has been rotated in its seat in the adapter housing 14 relative to the latter so that their respective center lines form an angle. This relative angular adjustment has been achieved in Fig. 6d by unscrewing the front pinning screw 22a almost completely from its hole 21 while the back screw has been screwed in to a corresponding extent. Thus the back screw 22b in the figure lifts the right part of the adapter head 8 which, because of the spheroidal external surface placed inside the sleeve part of the adapter housing, tips in the sleeve and rests at an angle to the left in the figure with its own center axis and the prosthesis collar attachment member center axis maximally angled to the left relative to the center axis of the adapter housing.

In Fig. 6e the situation is the reverse, that is, the front pinning screw 22a has been screwed in maximally while the back one 22b has been screwed out. The angle of the coincident center lines for the prosthesis collar attachment member 4 and the adapter head 8 relative to the center axis of the adapter head 8 is as great as is the case in Fig. 6d but in this case the center line of the prosthesis collar attachment member 4 and the adapter head 8 is angled to the right relative to the center line of the adapter housing.

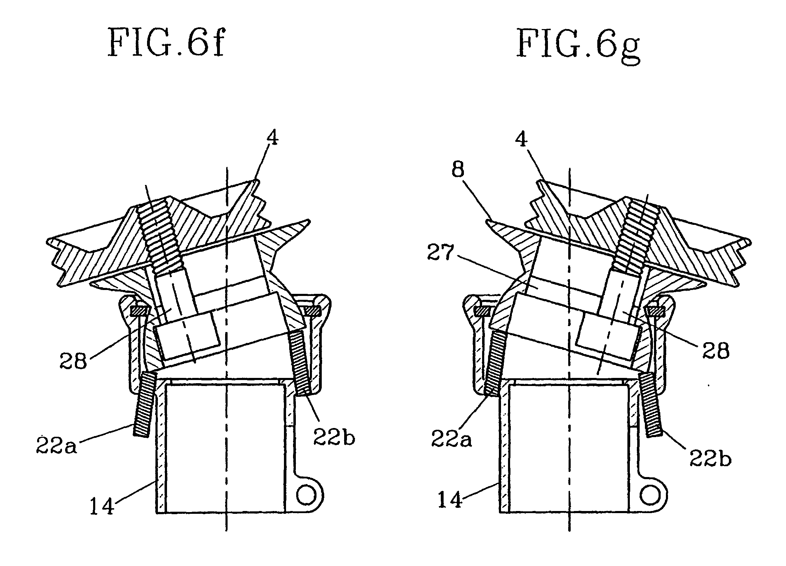

In Fig. 6f and 6g are finally shown two positions in which the adapter housing 14 and the adapter head 8 are angled relative to each other in the same way is in figures 6d and 6e where the angular adjustment has been made in the same way as in the two previously described cases. In addition the center axes of the prosthesis collar attachment member 4 and the adapter head 8 in the two figures 6f and 6g are shifted parallel relative to each other in the same way and by the same means as in figures 6a and 6b, respectively.

For simplicity in figures 6d to 6g how the adapter head 8 and the adapter housing 14 have been angled relative to each other has been shown only in the plane of the paper where only the front and back pinning screws 22a and 22b, respectively, have been screwed into or out from their neutral positions. Because the adapter housing 14 can also have more equidistant adjustment screws 22, preferably four, it is assumed that the angular adjustment can also be achieved in a plane perpendicular to the plane of the paper and/or in a combination of the planes, that is, the angle between the adapter housing and the adapter head, and thereby between the lower leg tube and the prosthesis collar attachment member 4, can be adjusted as desired to all angular positions while at the same time a sideways shift of the lower leg tube 2 and the prosthesis collar attachment member 4 can be obtained. Because the adapter head 8 has no fixed position relative to the adapter housing 14 the adapter head can also be rotated relative to the adapter housing 14 so that a parallel shift of the center axes of the prosthesis collar attachment member 4 and the adapter head 8 can be achieved in any desired direction perpendicular to the center axis of the adapter housing.

Using the design's large spheroidal adapter housing, which can be locked in position relative to the adapter housing in the way described and which has a built in sideways shift function, an extremely compact and therefore space saving design and a total adjustability for an externally applied knee/lower leg/foot prosthesis is achieved.

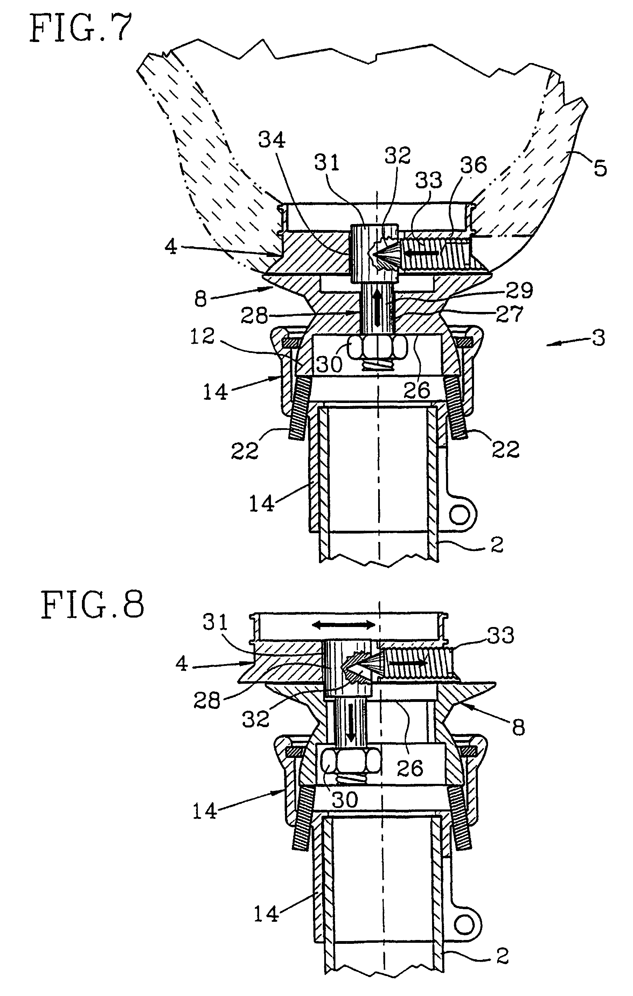

The above described sideways displacement function of the adapter is prevented by tightening the screw, which is accessible from inside the adapter housing 14. This means that the lower leg prosthesis 2 must be removed when an adjustment is to be made. According to the invention shown in Figs. 7 and 8 the adjustment can be made without needing to remove the lower leg prosthesis. For this purpose the connector 28, i.e. the screw is provided with a cylindric head 31, which is axially displaceable in a bore 34 in the prosthesis collar attachment means 4. The screw head 31 is provided with a radial conical recess 32, which with respect to the screw axle makes an inclined contact surface. In the prosthesis collar attachment means 4 there is arranged a manoeuvre member 33 in the form of a stop screw, the inner end of which is designed with a conical tip 35. This is intended to cooperate with the conical recess in such a way that when the stop screw is somewhat unscrewed from its threaded hole 36, the connector 28 can be axially displaced, so that the adapter housing 14 becomes moveable transversally with respect to the prosthesis collar attachment means, as can be seen from Fig. 8. After adjusting these parts relative to each other the stop screw 33 can be screwed into the conical recess in the locking member, at which the conical tip 35 cooperates with the inclined contact surface of the recess 32 and lifts the screw so much that the nut 30 will be brought in contact with the partition wall 26, as can be seen from Fig. 7.

The invention is not limited to the shown and described exemplifications but instead modifications are possible within the frame of the following patent claims.

## Claims

1. Adjustable adapter especially for a lower leg prosthesis including an adapter housing (14) attachable to the lower leg prosthesis (2), a spheroidal adapter head (8) mounted movably therein and adjustable by adjusting means (22) of the adapter housing, and a prosthesis collar attachment member (4) that can be shifted sideways relative to the adapter head (8), which latter is attachable to a prosthesis collar (5), and by means of a connector (28) is attachable to the adapter housing (14), at which the spheroidal part (12) of the adapter head (8), in mounted position, is integrated with the adapter housing (14) forming a unit assembled by two mutually movable parts which are fixable in different positions by means of the adjusting means (22), independantly of the adjustability and attachment of the adapter head to the prosthesis collar attachment member (4), at which in the adapter head (8) there is arranged an elongated through groove (27), through which said connector (28) is arranged to extend and which permits a transverse movement between the adapter head (8) and the prosthesis collar attachment member (4), independantly of the adapter housing (14) relative to the adapter head (8),
**characterized in,**
**that** the connector (28) is provided with an inclined contact surface (32), which is arranged to cooperate with a manoeuvre member (33) which is radially movable in the prosthesis collar attachment member (4), said manoeuvre member when being displaced in one direction is arranged to lift and in the opposite direction to sink the connector (28), at which a locking or releasing of the adapter head (8) against the prosthesis collar attachment member (4) is achieved, in order to allow said transverse movement between the adapter head (8) and the prosthesis collar attachment member (4).

2. Adapter according to claim 1,
characteriz ed in,
that the connector (28) is a screw (29) and a nut (30), and that said contact surface (32) is arranged in the head (31) of the screw, said contact surface having the shape of a conical recess.

## Patentansprüche

1. Verstellbarer Adapter, insbesondere für eine Unterschenkelprothese, einschließlich eines Adaptergehäuses (14), das an der Unterschenkelprothese (2) anbringbar ist, eines sphäroidischen Adapterkopfs (8), der beweglich im Adaptergehäuse montiert und durch Stellmittel (22) des Adaptergehäuses verstellbar ist, und eines Prothesenmanschetten-Befestigungsglieds (4), das relativ zum Adapterkopf (8) seitwärts verschiebbar ist, wobei Letzterer an einer Prothesenmanschette (5) und mit Hilfe eines Verbindungsstücks (28) am Adaptergehäuse (14) anbringbar ist, wobei der sphäroidische Teil (12) des Adapterkopfs (8) in montierter Stellung in das Adaptergehäuse (14) integriert ist und somit eine Einheit durch zwei gegenseitig bewegliche Teile gebildet wird, die mit Hilfe der Stellmittel (22) unabhängig von der Verstellbarkeit und der Befestigung des Adapterkopfs am Prothesenmanschetten-Befestigungsglied (4) in unterschiedlichen Stellungen feststellbar sind, wobei sich im Adapterkopf (8) ein durchgehendes Langloch (27) befindet, durch das sich das Verbindungsstück (28) erstreckt und das unabhängig von der Bewegung zwischen Adaptergehäuse (14) und Adapterkopf (8) eine Querbewegung zwischen dem Adapterkopf (8) und dem Prothesenmanschetten-Befestigungsglied (4) ermöglicht;
**dadurch gekennzeichnet,**
**dass** das Verbindungsstück (28) eine schräge Berührungsfläche (32) hat, die mit einem Stellglied (33) zusammenwirkt, das radial im Prothesenmanschetten-Befestigungsglied (4) beweglich ist und das bei einer Bewegung in die eine Richtung das Verbindungsstück (28) anhebt und bei einer Bewegung in die Gegenrichtung das Verbindungsstück (28) absenkt, wobei der Adapterkopf (8) mit dem Prothesenmanschetten-Befestigungsglied (4) verblockt bzw. von diesem gelöst wird, um die Querbewegung zwischen dem Adapterkopf (8) und dem Prothesenmanschetten-Befestigungsglied (4) zu ermöglichen.

2. Adapter nach Anspruch 1,
**dadurch gekennzeichnet,**
**dass** das Verbindungsstück (28) aus einer Schraube (29) und einer Mutter (30) besteht und dass sich die Berührungsfläche (32) im Kopf (31) der Schraube befindet, wobei die Berührungsfläche die Form einer konischen Vertiefung hat.

## Revendications

1. Adaptateur ajustable destiné en particulier à une prothèse de la partie inférieure de la jambe, comprenant un boîtier (14) d'adaptateur fixable à la prothèse (2) de la partie inférieure de la jambe, une tête (8) sphéroïdale d'adaptateur montée mobile à l'intérieur et réglable par des moyens de réglage (22) du boîtier d'adaptateur, et un élément (4) de fixation de l'emboîture de la prothèse, qui peut être décalé latéralement par rapport à la tête (8) de l'adaptateur, laquelle est fixable à une emboîture (5) de prothèse et, au moyen d'un connecteur (28), est fixable au boîtier (14) de l'adaptateur, adaptateur dans lequel la partie sphéroïdale (12) de la tête (8) d'adaptateur, en position montée, est intégrée avec le boîtier (14) d'adaptateur en formant une unité assemblée par deux parties mobiles l'une par rapport à l'autre qui sont fixables dans différentes positions au moyen des moyens de réglage (22), indépendamment de l'ajustabilité et de la fixation de la tête de l'adapteur à l'élément (4) de fixation de l'emboîture de prothèse, adaptateur dans lequel, dans la tête (8) de l'adaptateur est ménagée une gorge allongée traversante (27), à travers laquelle ledit connecteur (28) est placé pour s'étendre et qui permet un mouvement transversal entre la tête (8) de l'adaptateur et l'élément (4) de fixation de l'emboîture de prothèse, indépendamment du boîtier (14) de l'adaptateur par rapport à la tête (8) de l'adaptateur,
***caractérisé en ce que***
le connecteur (28) est muni d'une surface de contact inclinée (32), qui est prévue pour coopérer avec un élément de manoeuvre (33) qui est mobile radialement dans l'élément (4) de fixation de l'emboîture de prothèse, ledit élément de manoeuvre, lorsqu'il est déplacé dans une première direction, est prévu pour relever, et dans la direction opposée pour abaisser, le connecteur (28), un blocage ou une libération de la tête (8) d'adaptateur contre l'élément (4) de fixation de l'emboîture de prothèse étant ainsi obtenu(e), de manière à autoriser ledit mouvement transversal entre la tête (8) de l'adaptateur et l'élément (4) de fixation de l'emboîture de prothèse.

2. Adaptateur selon la revendication 1, ***caractérisé en ce que*** le connecteur (28) est une vis (29) et un écrou (30), et ***en ce que*** ladite surface de contact (32) est placée dans la tête (31) de la vis, ladite surface de contact ayant la forme d'un évidement conique.
